# EUROPEAN PATENT SPECIFICATION

(11) **EP 4 398 709 B1**
(45) Date of publication and mention of the grant of the patent: **02.07.2025**
(21) Application number: 22777365.2
(22) Date of filing: 25.08.2022
(51) Int. Cl.: A01G 18/10, A01G 18/40, A01G 7/00, A01G 29/00, C12N 1/14

(54) **USE OF NON-WOVEN FABRIC COMPRISING POLYLACTIC ACID (PLA) FOR THE INOCULATION OF PLANTS TO BE MYCORRHIZED**
VERWENDUNG VON VLIESSTOFF, DER POLYLAKTISCHE SÄURE (PLA) ENTHÄLT, ZUR INOKULATION VON PFLANZEN, DIE MYKORRHISIERT WERDEN
UTILISATION DE TISSU NON TISSE COMPRENANT DE L'ACIDE POLYLACTIQUE (PLA) POUR L'INOCULATION DE PLANTES A MYCORHISER

(30) Priority: 09.09.2021 IT 202100023342
(43) Date of publication of application: 17.07.2024
(73) Proprietor: Alma Mater Studiorum - Università di Bologna, 40126 Bologna (BO) (IT)
(72) Inventor: LEONARDI, Pamela, 82033 Cusano Mutri (BN) (IT); ZAMBONELLI, Alessandra, 40010 Sala Bolognese (BO) (IT); PULIGA, Federico, 40133 Bologna (BO) (IT)
(74) Representative: Fanfani, Stefano
(86) International application number: PCT/IT2022/050237
(87) International publication number: WO 2023/037393

(56) References cited:
- EP-A1- 2 591 904
- EP-A1- 3 521 511
- EP-A1- 3 812 495
- CN-U- 206 989 364
- CN-U- 211 881 365
- JP-A- 2011 193 797
- JP-A- 2015 019 585
- JP-A- 2021 040 521
- US-A1- 2018 092 311

## Description

### Technical field

The present invention belongs to the technical field of mycology, more specifically it belongs to the field of truffle colonization of mycorrhized plants.

In particular, the present invention relates to an innovative material for improving and standardizing inoculation techniques, both mycelial and sporal, in the field of truffle cultivation.

### Present status of the art

Truffle cultivation is now a part of agricultural production and many enterprises are approaching this culture. Modern truffle cultivation is based on planting plants that have been mycorrhized with truffles in nurseries; more precisely, it is based on the mycorrhizal symbiosis between the truffle plant and the species of the genus Tuber that is to be cultivated.

For cultivation to be successful, it is necessary for mycorrhized plants to be of excellent quality i.e., extensively colonized with truffle and free of pollutants.

For this reason, there is a need to standardize and improve the production of plants mycorrhized with truffles.

Mycorrhizae are part of a mutualistic symbiosis between a terricolous fungus (mycobiont) and the plant's root system (phytobiont), specifically the secondary roots.

The end result of the symbiosis is continuous nutritional exchanges between the two parties of this relationship: organic carbon is given up by the plant to the fungus while the latter provides the plant with water and important nutrients such as nitrogen, phosphorus, magnesium, zinc and copper. By protruding from the colonized roots, fungal mycelium provides the plant with a greater absorptive surface area (up to 1,000 meters of mycelium per meter of root) and a greater exploratory capacity of the portion of soil present around the root system. In the absence of such a symbiontic phenomenon, many plants would be in a perpetual state of stress and their development would suffer.

Truffles are ascomycete fungi belonging to several families of the order Pezizales, but only species of the genus Tuber are considered true truffles. The truffle is nothing more than the fruiting body (ascocarp or ascoma) of the fungus, the result of mycorrhizal symbiosis with shrub or tree plants.

Colonization of the plant's root system takes the form of numerous hyphal coiling around the root apex, which takes on its characteristic club shape, loses its absorptive hairs, and is stimulated by the fungus in the production of new root buds that are also subject to mycelial coiling, which continues its development by following the profile of the continuously changing root system.

The main mycorrhizal fungi used for food purposes are the following: *Amanita* spp., *Boletus* spp., *Cantharellus* spp., *Lactarius* spp., *Suillus* spp., *Tricholoma* spp., *Tuber* spp.

In the production of mycorrhized plants, the method of sporal inoculation was developed first, it remains the simplest method and at present it is still the most widely used, it involves the disintegration of the fruiting body of the truffle with the addition of sterile water to obtain a suspension of spores that is placed directly in contact with the root systems of plantlets raised on sterile substrates; according to the known technique, cultivation of the propagation material to be inoculated should be on inert substrates, such as lamellar flake vermiculite, agriperlite or the like, as described in CN105103947

In later times, the mycelial inoculation method began to spread, in which the mycelium of the fungal species of Tuber that is to be used for inoculation is isolated and grown on appropriate culture media until the required amount is obtained and then it is used to mycorrhize young plantlets micropropagated in vitro, cuttings or young seedlings.

Fungal mycelium can be obtained from fruiting body fragments, from ascospores in the germination stage, or from mycorrhizae in turn obtained from pure cultures; the use of pure cultures ensures that the inocula are free of undesirable fungi or contaminants that may inhibit root colonization of the target fungal species. Mycelia are able to colonize thinner roots more rapidly than spores, thus reducing the risk of contamination during plant growth.

It is a method that offers the same advantages as the previous one and also solves problems related to pollution by other fungal species. Moreover, in this way, being freed from the seasonality of nursery operations, it is possible to obtain mycorrhized plantlets at all times of the year. Patent application document JP2021040521 discloses a method using a cultivation material made of non-woven fabric carrying a specific fungus. Patent application documents JP2011193797 and JP2015019585 disclose different examples of a method of inoculating plants to be mycorrhized.

Currently, nursery enterprises producing truffle plants feel both the need to improve the results of sporal inoculation, currently in use in most nurseries, and to commercially introduce the technique of mycelial inoculation.

To improve the technique of spore inoculation and the contact of spores with plant roots, a method has been developed in which the spores are placed in contact with a cellulose-based non-woven fabric that is then wrapped around the roots to be mycorrhized; this technique allows for an increase in the surface area of the plant that is in contact with the spores, in contrast, the cellulose fabric takes a long time to degrade and disappear, and its mechanical characteristics hinder the development of the root system of the mycorrhized plantlet.

By way of non-limiting example, some of the most widely used symbionts for the production of mycorrhized plants, particularly with truffles, are: *Arbutus unedo* L., *Carya illinoensis* (Wangenh) K. Koch, *Cistus* spp., *Corylus* spp., *Fagus sylvatica* L., *Ostrya carpinifolia* Scop., Picea spp., *Pinus* spp., *Populus* spp., *Quercus* spp., *Salix* spp., *Tilia* spp.

### Objects and summary of the invention

It is therefore the object of the present invention to provide a technique for speeding up, simplifying and standardizing the production of sporal inoculum, while retaining the advantages shown by the use of cellulose fabric, but overcoming its disadvantages, with particular reference to slow biodegradability.

Further object of the present invention was to provide a method to employ a substrate that could ensure high and homogeneous mycelium development, in order to improve and standardize the mycelial inoculation technique.

Not least object of the present invention was to develop a method using a different material to increase the storage time of the mycelial inoculum.

These and other objectives that will become clear to the expert in the field were achieved by using polylactic acid (PLA)-based non-woven fabric to transfer the fungus to the root system of mycorrhized plantlets.

It should be noted that in the present specification, the term " non-woven fabric" of polylactic acid or, based on polylactic acid, means a non-woven fabric comprising polylactic acid.

Polylactic acid (PLA) is a bio-degradable aliphatic polyester derived completely from renewable resources and in agriculture it is mainly used in the production of biodegradable mulches.

Experimental results have confirmed that the use of polylactic acid-based non-woven fabric in truffle cultivation results in more extensively and uniformly mycorrhized plants than currently used techniques involving the use of vermiculite, or similar aggregates, as mycelium support and also compared with the use of cellulose fabric.

As a result, by adopting the polylactic acid-based non-woven fabric, mycorrhiza formation normally occurs within 50 days with the mycelial inoculation technique and within three months with the sporal inoculation technique, compared with the 3-4 months required with currently used techniques.

The polylactic acid-based non-woven fabric is able to hold the mycelium between its meshes, allowing more effective transfer of the fungus to the plants. Specifically, the mycelium develops between the loose fibers of the polylactic acid-based non-woven fabric, the latter of which is thin enough that it does not inhibit the development of plantlets' roots, which are able to break it down easily.

PLA non-woven fabric colonized by truffle mycelium can be stored at temperatures of 4°C for two months without altering its infectivity capabilities.

Using poly-lactic acid-based non-woven fabric considerably reduces the time to obtain mycorrhizae by mycelial inoculation (about 50 days). In fact, by using vermiculite or other inert materials for mycelium transfer, the time for obtaining mycorrhizae is about 90-120 days (3-4 months).

In addition to its rapid and complete biodegradability and the ease with which plant roots can pass through it, polylactic acid-based non-woven fabric has several other advantages, among which are the fact that it does not support microbial growth, is soft, lightweight and odorless, and comes from renewable sources.

In addition, polylactic acid-based non-woven fabric withstands high temperatures very well, without deforming and maintaining a loose, noncompact distribution of its fibers.

Experimental tests have also revealed that polylactic acid-based non-woven fabric, when immersed in a liquid, is able to attract fungal mycelium, causing it to adhere homogeneously over its entire surface.

Good results were obtained with PLA non-woven fabric with grammages (thicknesses) varying between 10 grams per square meter and 40 grams per square meter. The best results were obtained with grammages of 20 grams per square meter.

### Brief description of the drawings

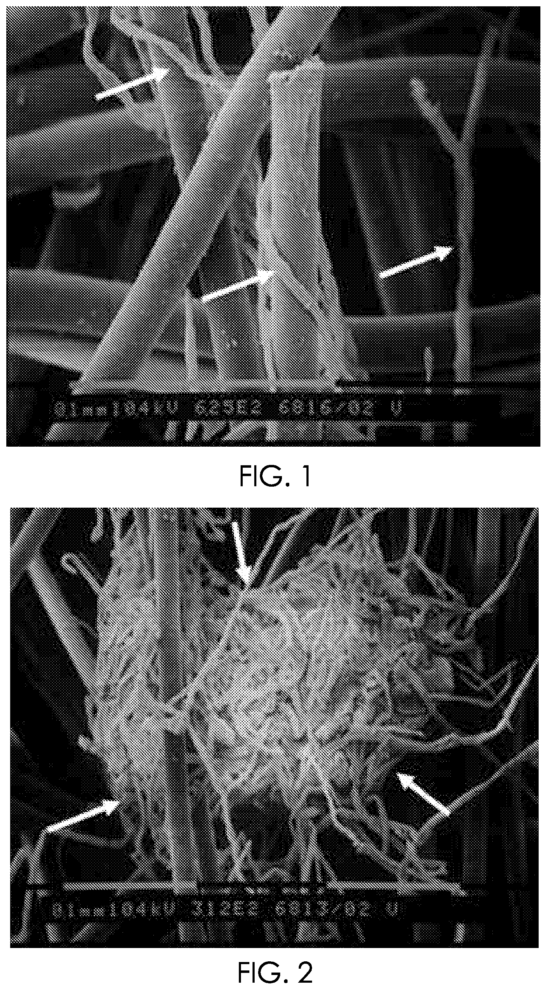

**Fig. 1** and **Fig. 2** show a scanning electron microscope (SEM) image portraying the mycelium of *Tuber borchii* intertwined with the fibers of PLA non-woven fabric, arrows indicate the mycelium.

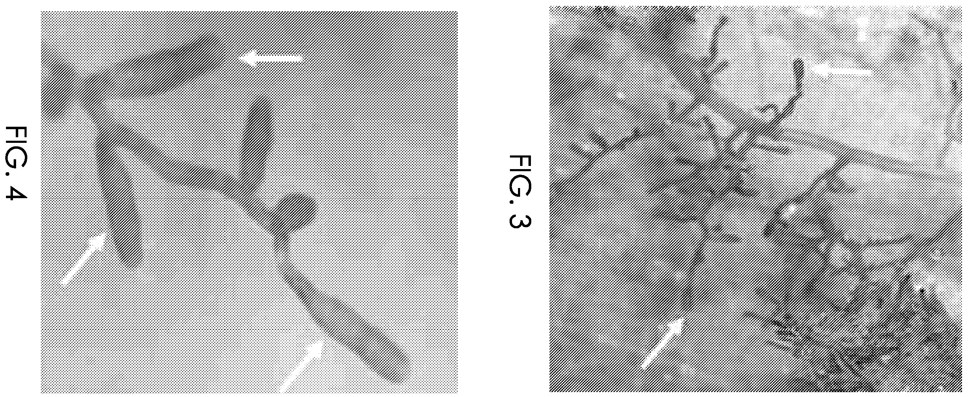

**Fig. 3** and **Fig. 4** show *Tuber borchii* mycorrhizae, indicated by arrows, observed under the stereomicroscope, related to the root systems of plants inoculated with PLA non-woven fabric.

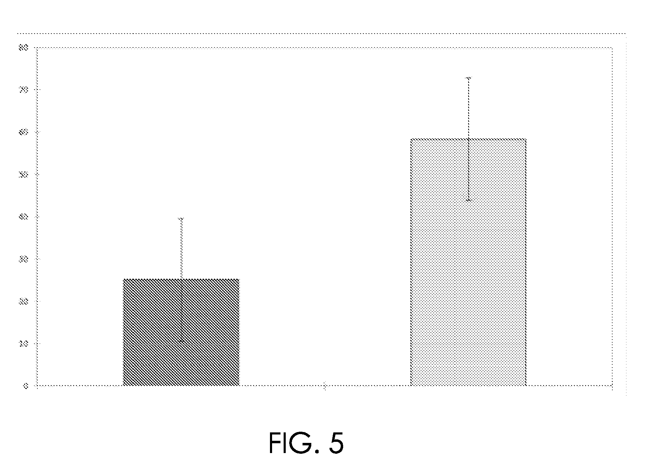

**Fig. 5** shows the different percentages of mycorrhization measured in a batch of oak plants that were comparatively evaluated. The left column shows the percentage of mycorrhization obtained with the classical mycelial inoculum, using vermiculite, while the right column shows the percentage of mycorrhization obtained using the polylactic acid-based non-woven fabric.

### Detailed description of two embodiments of the invention

According to a first implementation, polylactic acid-based non-woven fabric is employed by performing the sporal inoculation technique.

Preliminary disintegration of the truffle fruiting body can be done by different techniques and methods, usually with the use of a mortar or electric blender.

To facilitate the grinding of the fungal tissues, sterile water coupled with sand or other types of fine abrasive particles can be added to the crushing process.

The spore suspension thus obtained can be placed directly in contact with the non-woven polylactic acid-based fabric. The latter, after being so inoculated, must be wrapped around the root system of young sterile plantlets, which must then be grown in pots on sterile substrate for three to six months depending on the fungal species.

The spores, once germinated, develop a mycelium that will be trapped in the polylactic acid-based non-woven fabric and thus can better colonize the root system of the plantlets with which it is brought into contact.

In a second implementation, the polylactic acid-based non-woven fabric is employed by performing the mycelial inoculation technique, the latter depending on obtaining a pure mycelial culture of the fungus. The fungal mycelium can be obtained from fruiting body fragments, from ascospores in the germination stage, or from mycorrhizae that are themselves obtained from pure cultures.

The use of pure cultures ensures that the inocula are free of undesirable fungi or contaminants that may inhibit or supplant root colonization of the target fungal species. Mycelia are able to colonize thinner roots more rapidly than spores, thus reducing the risk of contamination during plant growth.

It should be emphasized that mycelial inoculation allows plants to be mycorrhized with fungal genotypes specifically selected for infectivity, affinity to the host plant, ecological conditions, and for qualitative and quantitative characteristics related to the fruiting body.

Most pure fungal cultures are obtained from fresh fruiting bodies, which offer less risk of contamination and require simpler isolation procedures. To isolate mycelium from fruiting bodies, small fragments of fungal tissue no more than one millimeter in length are taken from the gleba (hypogean fungi) or from the inner tissues of the intersection zone between the stem and the pileus (for epigeous fungi) and then transferred to agarized media.

Once the mycelium is obtained, it is then grown in Erlenmeyer flasks as a submerged culture in which pieces of polylactic acid-based non-woven fabric are inserted.

The culture medium may differ in composition depending on the fungal species. For *Tuber spp.* a liquid medium such as Woody Plant Medium (WPM) is recommended.

Mycelium in liquid culture is attracted to the PLA-based nonwoven fabric and develops its hyphae between the threads of the nonwoven fabric, as shown in the figures.

After a period varying between a minimum of one week and a maximum of two months, depending on the mycorrhizal fungus, the PLA-based non-woven fabric on which the mycelium has developed can be wrapped around the root systems of young plantlets, which must then be grown in pots on sterile substrate for two to four months, depending on the fungal species.

The large-scale nursery application of this type of inoculum is highly dependent on the potential of the fungal species of interest and their ability to produce large amounts of mycelium under axenic conditions.

For illustrative purposes, below is the typical composition of the modified Woody Plant Medium liquid substrate used with good results for the development of fungal mycelium of *Tuber spp.*

### Macroelements:

- 0, 5 g/l Ca (N_{O}3)₂·4H₂O
- 0, 2 g/l KH₂PO₄
- 0, 3 g/l MgSO₄·7H₂O
- 0, 1 g/l CaCl₂·2H₂O
- 0, 9 g/l K₂SO₄
- 0, 4 g/l NH₄NO₃

### Microelements:

- 0,014 g/l FeSO₄
- 1 ml/l di Na₂ EDTA (9,325g in 250ml of H₂O)
- 1 ml/l of micro solution (3,1 g/l H₃BO₃; 11,15 g/l MnSO₄ ·4H₂O; 4,3 g/l ZnSO₄·7H₂O; 0,0125 g/l CuSO₄·5H₂O; 0,125 g/l Na₂MoO₄·2H₂)

### Vitamins:

- asparagine 0,1g/l
- thiamine 0,1g/l
- biotin 0,01g/l

### Sugars:

- Glucose 5g/l
- Sucrose 5g/l
- myo-inositol 0,1g/l

## Claims

1. Method for inoculation of plants to be mycorrhized with spores comprising the following steps:
A) disintegration of a fruiting body of a fungus to obtain spores;
B) preparation of a suspension of said spores in sterile water;
C) inoculation of non-woven fabric comprising polylactic acid (PLA) fibers by imbibition in said spore suspension;
D) wrapping a root system of a plant to be mycorrhized with said soaked non-woven fabric.

2. Method for inoculation of plants to be mycorrhized **characterised by** comprising the step of:
A1) isolation of mycelium from a fruiting body of a mushroom;
B1) immersion of said mycelium in a culture medium in which a non-woven fabric comprising polylactic acid (PLA) fibers is also immersed;
C1) extracting said non-woven fabric from said culture medium and rinsing it;
D1) wrapping a root system of a plant to be mycorrhized with said non-woven fabric.

3. Method for inoculation of plants to be mycorrhized as per the preceding claim **characterized in that** said immersion phase B1 is prolonged for a period of time between 7 and 40 days, depending on the fungal species.

4. Method for inoculation of plants to be mycorrhized as per one of the preceding claims 2 to 3, **characterized in that** the root system of the plant to be mycorrhized is deprived of the tap-root before being wrapped in said non-woven fabric comprising polylactic acid (PLA).

5. Method for inoculation of plants to be mycorrhized as per the preceding claim **characterized in that** said culture medium comprises a Woody Plant Medium substrate.

## Patentansprüche

1. Verfahren zur Inokulation von Pflanzen, die mykorrhisiert werden sollen, bestehend aus den folgenden Schritten:
A) Zersetzung eines Fruchtkörpers eines Pilzes zur Gewinnung von Sporen;
B) Herstellung einer Suspension der genannten Sporen in sterilem Wasser;
C) Inokulation eines Vliesstoffs, der Polymilchsäure (PLA) -Fasern enthält, durch Imbibition mit der genannten Sporensuspension;
D) Umhüllen eines Wurzelsystems einer zu mykorrhizierenden Pflanze mit dem getränkten Vliesstoff.

2. Verfahren zur Inokulation von Pflanzen, die mykorrhiziert werden sollen, **dadurch gekennzeichnet, dass** es den folgenden Schritt umfasst:
A1) Isolierung von Myzel aus einem Fruchtkörper eines Pilzes;
B1) Eintauchen des Myzels in ein Kulturmedium, in das auch ein Vliesstoff, der Polymilchsäure (PLA) -Fasern enthält, eingetaucht wird;
C1) Herausnehmen des Vliesstoffs aus dem Kulturmedium und Spülen;
D) Umhüllen eines Wurzelsystems einer zu mykorrhizierenden Pflanze mit dem genannten Vliesstoff.

3. Verfahren zur Inokulation von Pflanzen, die mykorrhisiert werden sollen, nach dem vorhergehenden Anspruch, **dadurch gekennzeichnet, dass** die Immersionsphase B1 über einen Zeitraum zwischen 7 und 40 Tagen, je nach Pilzart, verlängert wird.

4. Verfahren zur Inokulation von Pflanzen, die mykorrhisiert werden sollen, nach einem der vorhergehenden Ansprüche 2 bis 3, **dadurch gekennzeichnet, dass** dem Wurzelsystem der zu mykorrhisierenden Pflanze die Pfahlwurzel entzogen wird, bevor es in den Vliesstoff, der Polymilchsäure (PLA) -Fasern enthält, eingewickelt wird.

5. Verfahren zur Inokulation von Pflanzen, die mykorrhisiert werden sollen, nach dem vorhergehenden Anspruch, **dadurch gekennzeichnet, dass** das genannte Kulturmedium ein "Woody Plant Medium" Substrat umfasst.

## Revendications

1. Procédé d'inoculation de plantes à mycorhizer à l'aide de spores, comprenant les étapes suivantes :
A) désintégration d'un sporophore d'un champignon afin d'obtenir des spores ;
B) préparation d'une suspension desdites spores dans de l'eau stérile ;
C) inoculation d'un tissu non tissé comprenant des fibres d'acide polylactique (PLA) par imbibition dans ladite suspension de spores ;
D) enrobage du système racinaire d'une plante à mycorhizer à l'aide dudit tissu non tissé imbibé.

2. Procédé d'inoculation de plantes à mycorhizer, **caractérisé en ce qu'**il comprend les étapes suivantes :
A1) isolement du mycélium à partir d'un sporophore d'un champignon ;
B1) immersion dudit mycélium dans un milieu de culture dans lequel est également immergé un tissu non tissé comprenant des fibres d'acide polylactique (PLA) ;
C1) extraction dudit tissu non tissé dudit milieu de culture et rinçage de celui-ci ;
D1) enrobage du système racinaire d'une plante à mycorhizer à l'aide dudit tissu non tissé.

3. Procédé d'inoculation de plantes à mycorhizer selon la revendication précédente, **caractérisé en ce que** ladite étape d'immersion B1 est prolongée pendant une durée comprise entre 7 et 40 jours, en fonction de l'espèce fongique.

4. Procédé d'inoculation de plantes à mycorhizer selon l'une des revendications 2 ou 3, **caractérisé en ce que** le système racinaire de la plante à mycorhizer est préalablement débarrassé de sa racine pivotante avant d'être enrobé dans ledit tissu non tissé comprenant de l'acide polylactique (PLA).

5. Procédé d'inoculation de plantes à mycorhizer selon la revendication précédente, **caractérisé en ce que** ledit milieu de culture comprend un substrat de type « Woody Plant Medium ».
